# EUROPEAN PATENT SPECIFICATION

(11) **EP 1 408 893 B1**
(45) Date of publication and mention of the grant of the patent: **12.01.2005**
(21) Application number: 02745622.7
(22) Date of filing: 15.07.2002
(51) Int. Cl.: A61F 5/01, A61F 13/08

(54) **AN INFLATABLE APPARATUS**
AUFBLASBARE VORRICHTUNG
APPAREIL GONFLABLE

(30) Priority: 20.07.2001 GB 0117707
(43) Date of publication of application: 21.04.2004
(73) Proprietor: HUNTLEIGH TECHNOLOGY PLC, Luton, Bedfordshire LU1 1TD (GB)
(72) Inventor: HAMPSON, David, Mark, Cardiff CF5 2QB (GB); SCHILD, Rolf, London N2 0BD (GB); WEBSTER, Nathan, Cardiff CF5 1RH (GB)
(74) Representative: Thaker, Shalini
(86) International application number: PCT/GB2002/003219
(87) International publication number: WO 2003/007855

(56) References cited:
- EP-A- 0 388 200
- WO-A-98/56331
- US-A- 2 724 129
- US-A- 3 826 249
- US-A- 4 300 759
- US-A- 4 841 956

## Description

The present invention relates to an inflatable apparatus, in particular, to a compression sleeve for use in general therapeutic treatment and the treatment of vascular disorders and edemas.

Prior to the present invention, various compression devices have been known in the art for applying compressive pressure to a patient's limbs in order to increase blood flow velocity.

It is known to have sequential compression devices providing intermittent pulses of compressed air which sequentially inflate multiple chambers in a sleeve to increase peak blood flow velocity, providing a non-invasive method of prophylaxis to reduce the incidence of deep vein thrombosis (DVT).

In general, these sequential compression devices of the prior art have a sleeve with a plurality of separate fluid pressure chambers progressively arranged longitudinally along the sleeve from a lower portion of the limb to an upper portion. Means are provided for intermittently forming a pressure pulse within these chambers from a source of pressurised fluid during periodic compression cycles. The sleeve may provide a compressive pressure gradient against the patient's limbs during these compression cycles which progressively decreases from the lower portion of the limb, e.g. from the ankle to the thigh.

Known multi-chamber compression sleeves have individual supply lines to each chamber. These supply lines lead back to a compressor unit and are linked using multiway connections. Each chamber's inflation and deflation is controlled by individual solenoid valves within the compressor unit.

However, these known systems suffer from the disadvantages of complexity of the number of parts required to control each chamber, the fit of the sleeve, and user problems in handling a bulky and complex tube and connector system attached to a sleeve.

The present invention seeks to provide a simpler, sequential compression system with a better fit and able to provide more control therapies. A compression sleeve according to the preamble of claim 1 is disclosed in WO 98 56331 A.

Accordingly, the invention provides a compression sleeve including a series of inflatable chambers, the first of the chambers connected to a fluid source, each of the chambers of the series interconnected so that the chambers inflate sequentially with respect to time and pressure, and control means to control the pressure profile of the chambers within the sleeve.

Preferably, each of the chambers in the series has means to exhaust to air to provide a pressure gradient between the chambers.

Preferably, the control means provides predetermined chamber rise times and set pressures by programming the power to the compressor to a predetermined algorithm.

The present invention due to the control and exhaust means in each chamber in series provides a sleeve deflation without a reverse pressure gradient on the patient limb unlike the prior art devices.

A preferred embodiment of the invention will now be described in detail, by way of example only, with reference to the accompanying drawings of which:
Figure 1 is a schematic diagram of the compression sleeve according to the invention;
Figure 2a is a schematic diagram of one embodiment of a compression sleeve according to the invention;
Figure 2b is a schematic diagram of a further embodiment of a compression sleeve according to the invention; and
Figure 3 is a graph showing the gradient sequential pressure rise against time achieved by the sleeves.

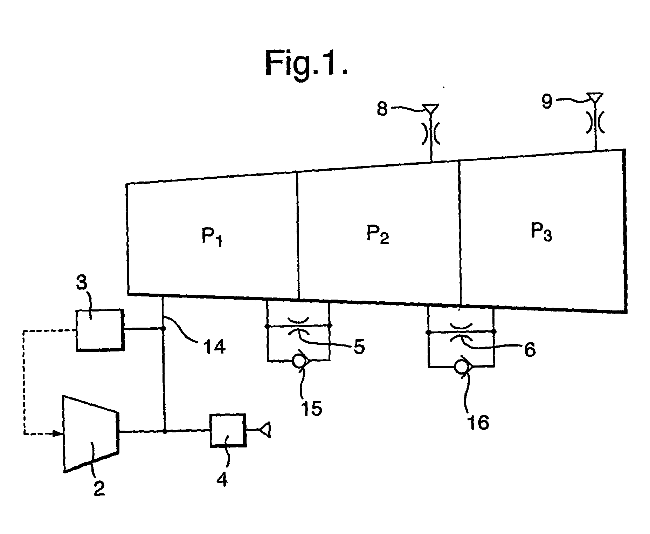

Referring to Figure 1, the sleeve 1 is supplied with air from a pump 2, which consists of a compressor driven by a closed loop control drive, having a feedback signal from a pressure transducer 3. The pump 2 also has a valve 4, which allows the air supply to be connected to atmosphere.

The sleeve 1 comprises two sheets of laminated materials. The inner layer consists of a breathable polyester foam laminated to an RF weldable backing of PVC.

The outer layer consists of loop pile fabric laminated to a RF weldable backing of PVC. They are placed together with the PVC layers together and welded to form a number of airtight chambers between the two layers.

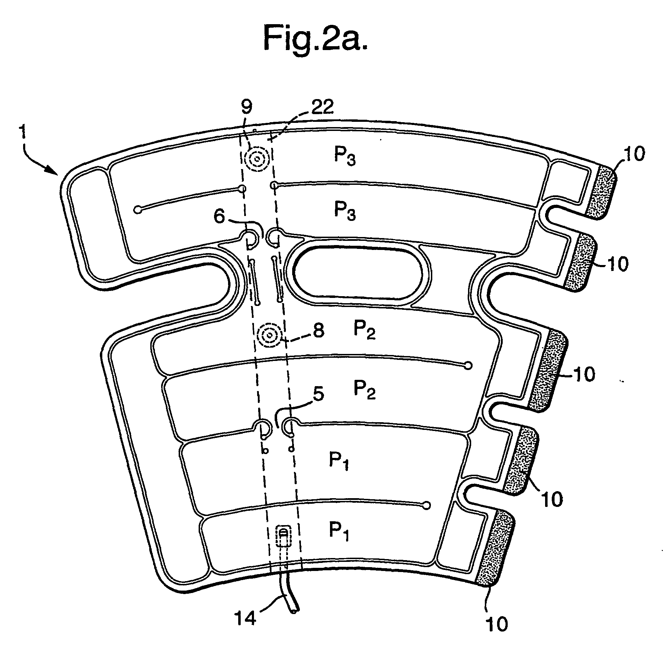

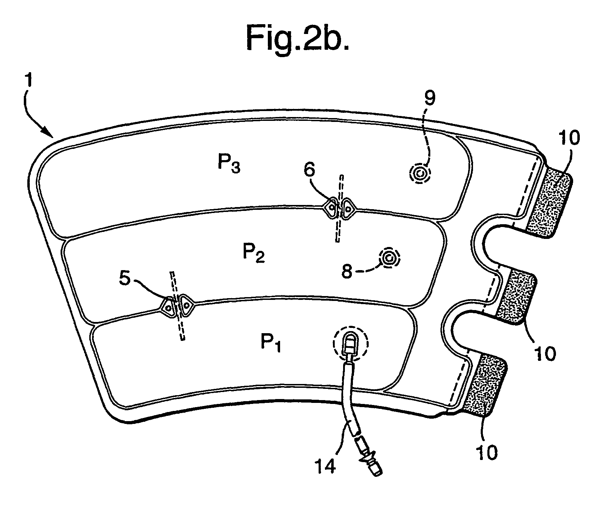

As shown in Figures 2a and 2b, the outside profile of the sleeve is shaped to fit around a patient's leg. The back of the leg is placed centrally in the bladder area of the sleeve and the two non-symmetrical sides brought around to wrap the sleeve around the leg.

The sleeve has hook pile tabs 10, along one edge, which engage with the outside loop pile fabric on the outside layer of the sleeve and secure the sleeve in place on the leg.

The sleeve is sub-divided into three chambers, P1, P2 and P3 arranged longitudinally along the sleeve to enclose a leg from ankle to thigh.

When positioned around a leg, chamber P1 will apply compressive pressure to the ankle region; chamber P2 to the calf region; and chamber P3 will apply compressive pressure to the thigh region. Figure 2a shows the chambers P1, P2, P3 each separated into lower and upper sections respectively. Figure 2b, shows the chambers P1, P2, P3 as single chambers without any separation.

Restrictors 5 and 6 interconnect the chambers P1, P2 and P3. The restrictors may be valves, bleed gaps in the weld lines or thin PVC tube of sufficient length to provide the required air flow restriction. Chambers P2 and P3 also have orifices 8 and 9 of a predetermined size for exhaust of air to atmosphere. Chamber P1 is provided with a conduit 14 connecting chamber P1 to the pump 2.

When compressed air is supplied to chamber P1 by conduit 14, the chamber will start to inflate and apply pressure to the enclosed limb. As the sleeve is stretched around the leg, it mechanically holds the two layers together. The air pressure progressively opens the bladder as it inflates its two sections, which in combination with the restrictor 5, imparts a time delay on inflating the chamber along the inflation path.

The compressed air then bleeds through into chamber P2, via restrictor 5. Chamber P2 progressively inflates in the same manner and air bleeds into chamber P3 via restrictor 6.

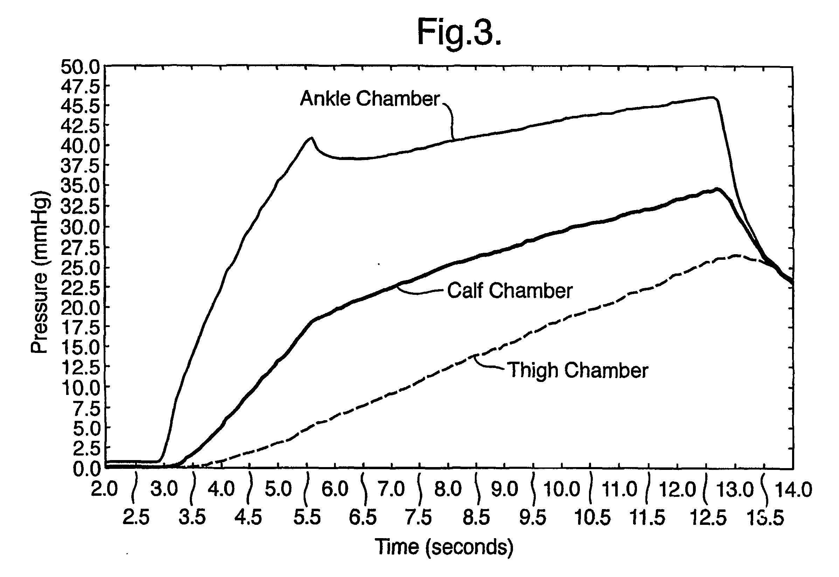

On initial inflation, the compressor is driven by closed loop control to maintain a pre-determined pressure rise and hold pressure in chamber P1 as shown in Figure 3. The compressed air inflates the subsequent chambers P2, P3 through the interchamber restrictors 5 and 6, giving a sequential rise in pressure to each chamber P2 and P3. The particular "shepherd's crook" shape to the restrictors 5, 6 profile, is an anti-kink device. As each chamber inflates, it also inflates the circular portion within the "crook". This stiffens the walls each side of the restrictors and helps to keep it open for the passage of air.

The orifices 8 and 9 to atmosphere, control the leak rate out of each chamber P2, P3 and are sized to give a predetermined pressure gradient between chambers P1, P2, P3 (see Figure 3). By this means, the pressure will be lower in the successive chambers P1, P2 and P3, varying the pressure from ankle to calf to thigh. Alternately, one way valves 15, 16 provide a means of rapid deflation of the chambers if required.

These physical features in the sleeve, coupled with a pre-programmed air delivery from the compressor, result in a three-chamber sleeve that inflates the chambers sequentially in time and with a decreasing pressure gradient between the distal chamber P1 through to the proximal chamber P3.

Also, instead of the PVC tubing, a strip of air permeable open cell polyester foam 22 may be welded between the two layers. The foam 22 helps to keep the narrow air passages 5, 6 open and prevents blocking due to sleeve folds which may occur during use on a patient's leg.

Apart from the rapid deflation, in normal use, the deflation of the chambers is achieved, by firstly switching off the compressor, holding the pressure in the distal chamber P1 and allowing the pressure to decrease in the subsequent chambers P2, P3 through the orifices 8 and 9 to atmosphere. Then, at a predetermined time, the exhaust valve 4 in the pump 2 is opened, allowing the distal chamber P1 to deflate through the supply conduit 14 after the other chambers P2, P3.

This two-stage deflation helps prevent a reverse pressure gradient on the user's leg, which is considered to be clinically detrimental.

## Claims

1. A compression sleeve (1) comprising a series of inflatable chambers (P1, P2, P3), the first (P1) of the chambers connected to a fluid source (2) each of the chambers of the series interconnected so that the chambers inflate sequentially with respect to time and pressure, and control means to control the inflation pressure and time in each of the chambers, **characterised in that** the control means is located within the sleeve.

2. A compression sleeve as claimed in claim 1 **characterised in that** each of the chambers in the series has means (8, 9) to exhaust to air.

3. A compression sleeve as claimed in claim 1 or 2 **characterised in that** the chambers are interconnected to each other by restrictors.

4. A compression sleeve as claimed in claim 3 **characterised in that** the restrictor includes a thin inflexible tube of sufficient length to give the required air flow restriction.

5. A compression sleeve as claimed in claim 1, 2 or 3 **characterised in that** an anti-kink device comprises the restrictor having a "shepherd's crook" shape.

## Patentansprüche

1. Kompressionshülle (1) mit einer Reihe von aufblasbaren Kammem (P1, P2, P3), wobei die erste Kammer (P1) an eine Fluidquelle (2) angeschlossen ist, die Kammern der Reihe so miteinander verbunden sind, dass die Kammern bezogen auf Zeit und Druck aufeinanderfolgend aufgeblasen werden, und Steuermitteln zum Steuern des Aufblasdruckes und der Aufblaszeit in jeder der Kammern,
**dadurch gekennzeichnet, dass** die Steuermittel innerhalb der Hülle angeordnet sind.

2. Kompressionshülle nach Anspruch 1,
**dadurch gekennzeichnet, dass** jede Kammer der Reihe Mittel (8, 9) zum Ablassen von Luft hat.

3. Kompressionshülle nach Anspruch 1 oder 2,
**dadurch gekennzeichnet, dass** die Kammern mit Reduzierstücken miteinander verbunden sind.

4. Kompressionshülle nach Anspruch 3,
**dadurch gekennzeichnet, dass** das Reduzierstück ein dünnes, nicht flexibles Rohr mit ausreichender Länge hat, um die erforderliche Luftströmungsreduzierung zu erzeugen.

5. Kompressionshülle nach Anspruch 1,2 oder 3,
**dadurch gekennzeichnet, dass** eine Knickschutzvorrichtung das Reduzierstück aufweist, welches die Form eines "Hirtenstabs" hat.

## Revendications

1. Brassard de compression (1) comprenant une série de chambres gonflables (P1, P2, P3), la première (P1) des chambres étant reliée à une source de fluide (2), chacune des chambres de la série étant reliée mutuellement de sorte que les chambres gonflent séquentiellement par rapport au temps et à la pression, et un moyen de commande pour commander la pression de gonflage et le temps dans chacune des chambres, **caractérisé en ce que** le moyen de commande est situé à l'intérieur du brassard.

2. Brassard de compression selon la revendication 1, **caractérisé en ce que** chacune des chambres de la série comporte un moyen (8, 9) pour évacuation à l'air.

3. Brassard de compression selon la revendication 1 ou 2, **caractérisé en ce que** les chambres sont reliées mutuellement l'une à l'autre par des éléments de restriction.

4. Brassard de compression selon la revendication 3, **caractérisé en ce que** l'élément de restriction comprend un tube non flexible mince de longueur suffisante pour conférer la restriction de débit d'air requise.

5. Brassard de compression selon la revendication 1, 2 ou 3, **caractérisé en ce qu'**un dispositif anti-vrillage comprend l'élément de restriction présentant une forme de "crochet de berger".
